# EUROPEAN PATENT SPECIFICATION

(11) **EP 0 973 558 B1**
(45) Date of publication and mention of the grant of the patent: **22.09.2004**
(21) Application number: 98901899.9
(22) Date of filing: 02.02.1998
(51) Int. Cl.: A61L 24/04, A61P 23/00, A61K 6/08, A61K 31/167

(54) **ANAESTHETIC BONE CEMENT**
ANÄSTEHISCHER KNOCHENZEMENT
CIMENT OSSEUX ANESTHESIANT

(30) Priority: 07.02.1997 US 799007; 01.04.1997 US 825943
(43) Date of publication of application: 26.01.2000
(73) Proprietor: QUEEN'S UNIVERSITY AT KINGSTON, Kingston Ontario K7L 3N6 (CA)
(72) Inventor: BOND, David, M., Kingston, Ontario K0H 1S0 (CA); RUDAN, John, F., Kingston, Ontario K7L 4V1 (CA); ADAMS, Michael, A., Kingston, Ontario K7M 5X9 (CA)
(74) Representative: Simpson, Alison Elizabeth Fraser
(86) International application number: PCT/CA1998/000060
(87) International publication number: WO 1998/034653

(56) References cited:
- EP-A- 0 301 759
- WO-A-88/05650
- US-A- 4 005 191
- CHEMICAL ABSTRACTS, vol. 108, no. 16, 18 April 1988 Columbus, Ohio, US; abstract no. 137849, GRUNTOVA Z ET AL: "BIOADHESIVE PREPARATIONS WITH LOCAL ANESTHETIC AGENTS FOR DENTAL USE" XP002068850 & FARM. FAK., UNIV. KOMENSKEHO, BRATILAVA, vol. 36, no. 9, 1987, CZECH., pages 395-400,
- PATENT ABSTRACTS OF JAPAN vol. 015, no. 133 (C-0820), 2 April 1991 & JP 03 017003 A (SANKIN KOGYO KK), 25 January 1991,

## Description

### CROSS-REFERENCE TO RELATED APPLICATIONS

This application is a continuation-in-part of U.S. Patent Application No. 08/799,007, filed February 2, 1997, and U.S. Patent Application No. 08/825,943, filed April 1, 1997.

### FIELD OF THE INVENTION

This invention relates to surgical bone cement compositions and more particularly to bone cement compositions having anaesthetic properties, and for producing analgesia.

### BACKGROUND OF THE INVENTION

Polymer based surgical bone cements have been used for many years to fill voids in bones and to improve fixation of implanted orthopaedic prosthetic devices. Typically such cements contain polymers or copolymers of alkyl methacrylate and/or copolymers of methyl methacrylate with methyl acrylate or styrene. The liquid compound consisting of esters of acrylic or methacrylic acid (typically methyl methacrylate) is packaged in an ampoule, possibly with additives such as premature polymerization preventers such as hydroquinone, and curing promoters such as N, N-dimethyl-p-toluidine. A polymerization initiator, typically an organic peroxy compound such as powdered benzoyl peroxide, is combined with the polymeric component and a radiopacifier (such as barium sulphate or zirconium dioxide) for rendering the bone cement opaque to X-rays. The polymeric materials are generally sterilized by either irradiation or gas sterilization. In use, typically a bone is cut and prepared to receive a surgical implant and then the liquid and dry components of the cement, contained in the ampoule and powder bag are mixed together to form a paste which can then be applied by the surgeon to the cut bone. The implant can then be set in the paste which, when fully polymerized, forms a continuous solid interface between the implant and the bone.

It is also known to incorporate therapeutic or diagnostic substances into the bone cement for various purposes. For example, U.S. Patent No. 4,900,546, issued 13 February 1990, to Poseyn Dowty *et al*., teaches the incorporation of antibiotics such as gentamycin, penicillin and tetracycline; anti-cancer drugs; anti-inflammatory drugs; immuno-stimulants; immuno-suppressants; osteogenic promoters and diagnostic substances such as radioactive tracers. While some anti-inflammatory drugs may have analgesic properties, such compounds are not anaesthetics.

Although local anaesthetics, such as lidocaine and prilocaine are known to have potent anti-microbial activity (anti-bacterial and anti-fungal), when used in relatively high dosages (0.5-2% solution) (*J. Infect. Diseases,* 121:597-607, 1970), heretofore such anaesthetic compounds have not been incorporated into bone cements for the promotion of anaesthesia. It has now been found that substantial pain relief can be achieved by incorporating into a known bone cement composition a local anaesthetic at a dosage level several orders of magnitude lower than would be required to achieve an anti-microbial effect with such anaesthetic.

### OBJECT OF THE INVENTION

An object of the present invention is to provide novel bone cement compositions which incorporate an anaesthetic, and have analgesic properties. Another object of this invention is to provide composition for use for producing analgesia in the vicinity of a bone surgery site.

### SUMMARY OF THE INVENTION

By a broad aspect of this invention, there is provided an anaesthetic bone cement comprising a bone cement composition including an effective amount up to about 5% by weight of an anaesthetic, preferably a local anaesthetic.

By a preferred aspect of this invention, there is provided an anaesthetic bone cement composition comprising: (a) a liquid monomeric (meth)acrylate composition; (b) a powder comprising at least one of a homopolymer and a copolymer of methyl methacrylate containing an effective amount of a polymerization initiator and a radiopacifier; and (c) an effective amount up to about 5% by weight of said bone cement composition of a local anaesthetic.

An anaesthetic bone cement composition in accordance with the invention includes a local anaesthetic in a physical form such as, for example, liquids and solids, and a chemical form such as, for example, acids and bases. A local anaesthetic is released from the bone cement in a preselected characteristic release profile that is determined by at least one of chemical form and physical form of the anaesthetic. An anaesthetic bone cement composition according to the invention comprises an anaesthetic in an amount between about 0.007% and about 5% by weight of said cement composition. An anaesthetic bone cement composition according to the invention can comprise a local anaesthetic provided in at least two different forms, the forms being, for example, acids, bases, solids, and liquids. Also in accordance with the invention, an anaesthetic bone cement composition can comprises a combination of at least two anaesthetics selected from the group consisting of lidocaine, bupivacaine, prilocaine and tetracaine. When a combination of two or more different anaesthetics are included in the bone cement composition of the invention, they can be in two different forms, the forms being selected from the group consisting of acids, bases, solids, and liquids, or they can be of the same form.

By another aspect of this invention, there is provided a process for producing an anaesthetic bone cement comprising combining: (a) a liquid monomeric (meth)acrylate; (b) a powdered component comprising at least one of a homopolymer and a copolymer of methyl methacrylate, an effective amount of a polymerization initiator and a radiopacifier; and (c) an effective amount up to about 5% by weight of a local anaesthetic.

By yet another aspect of this invention there is provided the anaesthetic bone cement composition for producing analgesia at an orthopaedic implant site in a patient, comprising cutting and preparing a bone at said site to receive said implant and applying to said prepared bone a bone cement composition comprising: (a) a liquid monomeric (meth)acrylate composition; (b) a powder comprising at least one of a homopolymer and a copolymer of methyl methacrylate containing an effective amount of a polymerization initiator and a radiopacifier; and (c) an effective amount up to about 5% by weight of said bone cement composition of a local anaesthetic.

According to the use of the invention, a local anaesthetic included in a bone cement composition can be in a physical form such as, for example, liquids and solids, and a chemical form such as, for example, acids and bases. A local anaesthetic is released from the bone cement in a preselected characteristic release profile that is determined by at least one of chemical form and physical form of the anaesthetic. An anaesthetic bone cement composition according to the method of the invention comprises an anaesthetic in an amount between about 0.007% and about 5% by weight of said cement composition. An anaesthetic bone cement composition according to the use of the invention can comprise a local anaesthetic provided in at least two different forms, the forms being, for example, acids, bases, solids, and liquids. Also in accordance with the use of the invention, an anaesthetic bone cement composition can comprises a combination of at least two anaesthetics selected from the group consisting of lidocaine, bupivacaine, prilocaine and tetracaine. When the use of the invention involves use of a combination of two or more different anaesthetics, they can be in two different forms, the forms being selected from the group consisting of acids, bases, solids, and liquids, or they can be of the same form.

### BRIEF DESCRIPTION OF THE DRAWINGS

Figure 1 is a graph showing release of lidocaine from Howmedica bone cement;
Figure 2 is a graph showing release of lidocaine from Zimmer bone cement;
Figure 3 is a graph showing release of lidocaine from DePuy CMW3 bone cement;
Figure 4 is a graph showing release profiles of crystalline and liquid lidocaine and prilocaine base from DePuy CMW3 bone cement;
Figure 5 is a graph showing release profiles of crystalline and liquid lidocaine HCl from DePuy CMW3 bone cement; and
Figure 6 is a graph showing release profiles of crystalline lidocaine base and crystalline lidocaine HCl from DePuy CMW3 bone cement.

### DETAILED DESCRIPTION OF THE INVENTION

By one aspect, this invention pertains to compositions comprising bone cement and an anaesthetic, preferably a local anaesthetic. The compositions of the invention provide a means by which the anaesthetic can be delivered precisely to the site where it is required, for reduction or elimination of pain, simultaneously with the application of bone cement. Compositions of the invention are useful in, but not limited to, surgical procedures involving, for example, manipulation, repair, and replacement of bone and of joints between bones. A typical example of a procedure in which compositions of the invention may be employed is hip replacement surgery. Compositions according to the invention are therefore also useful in procedures in which prosthetic devices or orthopaedic implants are attached to bone. In general, therefore, the compositions of the invention may be employed in any surgical procedure in which bone cement is employed and analgesia is desired.

One advantage of the compositions according to the invention is that the combination of bone cement and an anaesthetic does not result in any reduction in efficacy of the anaesthetic or of the bone cement. Moreover, the combination of bone cement and anaesthetic according to the invention provides a sustained release of the anaesthetic over a prolonged period such as, for example, several days, eliminating the need for repeated administration of anaesthetic. A further advantage of the compositions of the invention is that delivery of the anaesthetic precisely to the source of pain permits smaller doses of anaesthetic to be used, relative to conventional modes of administration such as intramuscular injection. Further, local delivery of the anaesthetic to the site where it is required may provide enhanced efficacy.

Bone cements suitable for use in the compositions of the invention include any commercially available bone cements generally comprising a liquid monomeric (meth)acrylate composition and a powder comprising at least one of a homopolymer and a copolymer of methyl methacrylate containing an effective amount of a polymerization initiator and a radiopacifier. Suitable bone cements are, for example, Howmedica Simplex® (Limerick, Ireland), Zimmer Osteobond™ (Warsaw, Indiana), and DePuy® CMW3™ and CMW Endurance™ (Warsaw, Indiana). DePuy CMW3 is a preferred bone cement. However, the invention is not limited to these bone cements and others may be equally suitable.

Anaesthetics suitable for use in the compositions of the invention are, for example, lidocaine, bupivacaine, prilocaine (amide family), and tetracaine (ester family). However, the invention is not limited to these anaesthetics and others may be equally suitable. A preferred anaesthetic is Xylocaine® (Astra Pharmaceuticals, Sodertalje, Sweden) brand of lidocaine. The compositions of the invention contain an effective amount of the anaesthetic, which amount is up to about 5% by weight of the bone cement composition. In some cases, the anaesthetic incorporated in the bone cement composition may be provided in a precursor (or "prodrug") form which is converted *in vivo* to an anaesthetic that produces analgesia according to the invention. (See R.B. Silverman, *The Organic Chemistry of Drug Design and Drug Action,* Academic Press, Chapter 8, 1992.) Prodrugs (for example, an ester which is hydrolyzed *in vivo*) are employed in the art to alter biodistribution of an active compound or to modify metabolic or kinetic properties thereof.

As used herein, the term "effective amount" means an amount of anaesthetic required to achieve the desired result of reduction or elimination of pain.

As used herein, the term "anaesthetic" refers to a compound that reversibly depresses neuronal function, producing loss of ability to perceive pain or other sensations.

As used herein, the term "local anaesthetic" refers to a compound that reversibly depresses neuronal function, producing loss of ability to perceive pain or other sensations, the local anaesthetic acting locally, i.e., at the site or vicinity of its application, to prevent perception of pain.

Anaesthetics are generally amides or ester compounds which depress neuronal function and thus deaden or block pain by inhibiting the neural transmission of pain signals. Anaesthetics are therefore different from NSAID analgesic compounds such as aspirin or acetaminophen which act in an entirely different manner to provide analgesia but not anaesthesia.

As used herein, the term "analgesia" refers to a neurological or pharmacological state characterized by an absence of normal sensibility to pain, without an effect on consciousness. Accordingly, painful stimuli are either not perceived at all, or they are moderated such that, even though they may still be perceived, they are no longer painful.

The present invention is concerned with the use of local anaesthetics, such as lidocaine, bupivacaine, prilocaine (amide family), and tetracaine (ester family) to provide analgesia in body tissues surrounding a surgical site in which a bone cement has been employed.

By another aspect, this invention pertains to a process for producing an anaesthetic bone cement composition comprising combining a commercially available bone cement such as, but not limited to, Howmedica Simplex, Zimmer Osteobond, and DePuy CMW3 and CMW Endurance, with an effective amount up to about 5% by weight of a local anaesthetic. Bone cements suitable for use in the process of the invention generally comprise a liquid monomeric (meth)acrylate composition and a powder comprising at least one of a homopolymer and a copolymer of methyl methacrylate containing an effective amount of a polymerization initiator and a radiopacifier. Examples of anaesthetics suitable for use in the process of the invention are lidocaine, bupivacaine, prilocaine (amide family), and tetracaine (ester family), and others may be equally suitable.

By yet another aspect, this invention pertains to a composition for use for producing analgesia in a patient at the site of a surgical procedure involving manipulating, repairing, and/or replacing bone and joints between bones, and including, for example, implanting prosthetic or orthopaedic devices. The method of the invention comprises preparing bones at the site and applying to the prepared bones a bone cement composition comprising a liquid monomeric (meth)acrylate composition, a powder comprising at least one of a homopolymer and a copolymer of methyl methacrylate containing an effective amount of a polymerization initiator and a radiopacifier; and an effective amount up to about 5% by weight of the bone cement composition of an anaesthetic. Bone cements suitable for use in the method of the invention include, but are not limited to, commercially available bone cements such as Howmedica Simplex, Zimmer Osteobond, and DePuy CMW3 and CMW Endurance. Anaesthetics suitable for use in the method of the invention include lidocaine, bupivacaine, prilocaine (amide family), and tetracaine (ester family), and others may be equally suitable.

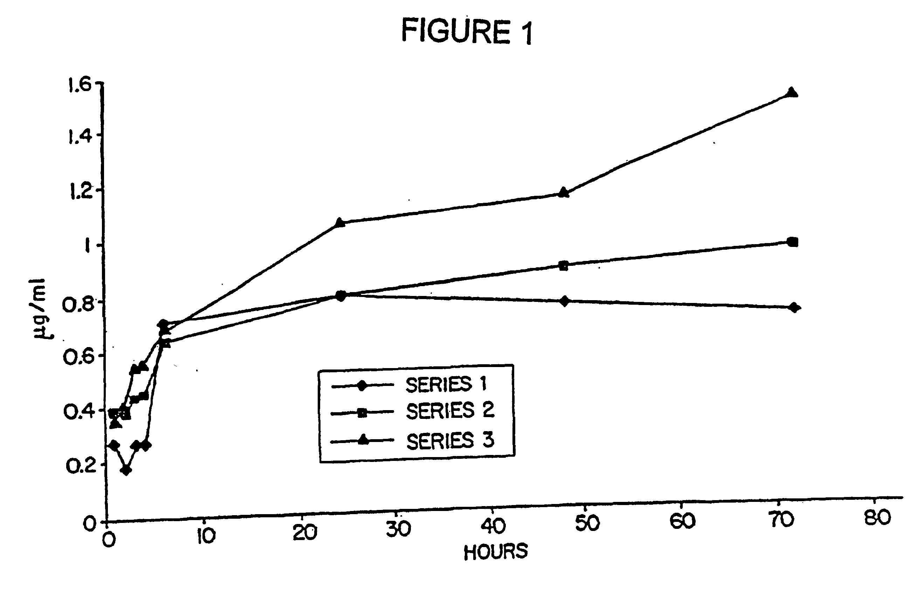

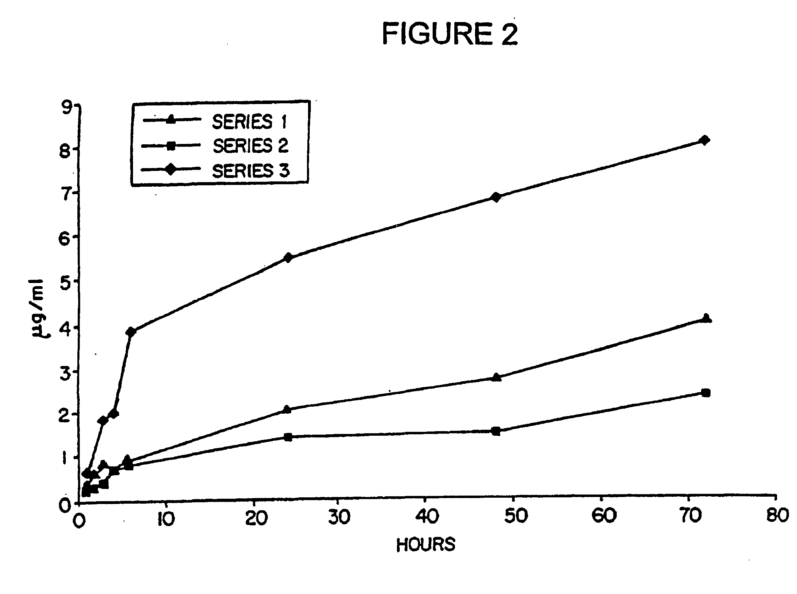

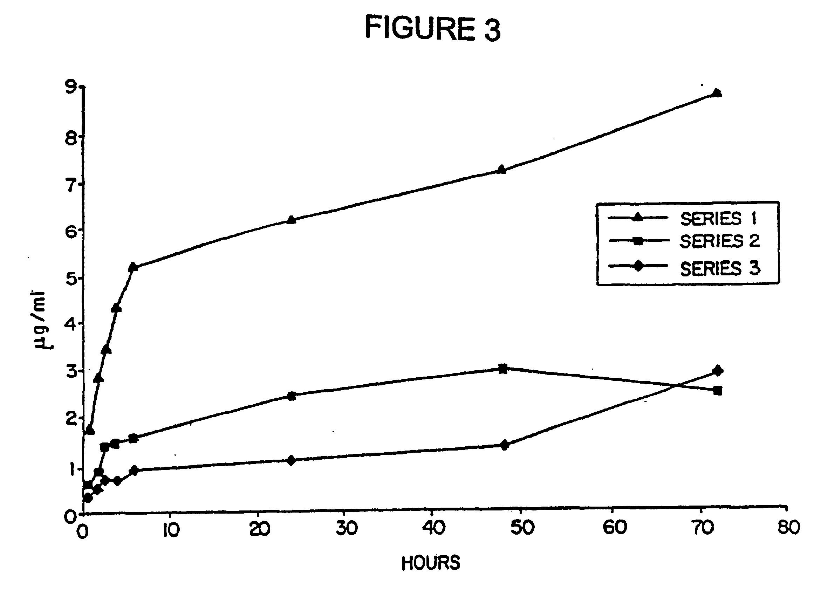

To determine whether local anaesthetics elute from a bone cement containing from about 2.0% to about 5.0% by weight of the anaesthetic, a series of elution studies were performed in which lidocaine (Xylocaine, Astra Pharmaceuticals) was combined with Howmedica, Zimmer, and DePuy bone cement (see Example 1). The elution profiles shown in Figures 1, 2, and 3 indicate that this form of lidocaine is released from the bone cement mixture in an amount proportional to the amount of lidocaine in the mixture. The elution profiles further indicate that adequate amounts of lidocaine are released from bone cement to provide the desired efficacy and hence that concentrations of the anaesthetic less than 2% by weight of the bone cement also would provide the desired efficacy.

Anaesthetics suitable for use according to the invention are available as liquids and solids, wherein the solid form is a crystalline powder. In addition, anaesthetics such as lidocaine are also available in base or acid (HCl) forms. Lidocaine used in the studies of Example 1 was crystalline lidocaine base, whereas the studies described in Example 2 concern the release characteristics of alternate forms of anaesthetics when combined with bone cement.

The studies of Example 2 employed crystalline lidocaine base from three manufacturers (Sigma, St. Louis, MO; Spectrum, Gardena, CA; and Wyckoff, South Haven, MI) and Depuy CMW3 and CMW Endurance bone cement. Studies examined the effects of manufacturer of anaesthetic, type of anaesthetic (i.e., lidocaine vs. prilocaine), physical form (i.e., crystalline vs. liquid), particle size of crystalline anaesthetic, type of bone cement (CMW3 vs. CMW Endurance), concentration of anaesthetic (about 0.008 % to about 3.5% by weight of bone cement), and chemical form (base vs. HCl), on release profiles (i.e., the amount of anaesthetic released after a given period and the change in rate of elution of anaesthetic over time).

The studies of Example 2 demonstrate that lidocaine currently available from three different manufacturers has the same release characteristics when combined with bone cement according to the invention. In addition, although crystalline forms of anaesthetic from different manufacturers may vary with respect to the proportions of different-sized particles present, particle size does not affect the overall release of anaesthetic after a period of 72 hours. However, as smaller particles of anaesthetic elute faster than larger particles, particularly in the case of lidocaine HCl, particle size should be considered in preparing compositions of the invention when specific elution profiles are desired. For example, by incorporating primarily smaller particles (e.g., 75 to 150 um, see Example 2), a bone cement composition having faster initial release of anaesthetic, and a relatively shorter overall period of release, can be prepared. Conversely, by incorporating primarily larger particles (e.g., > 250 um), a bone cement composition having slower initial release of anaesthetic, and a relatively longer overall period of release, can be prepared. Thus it can be seen how anaesthetic bone cement compositions according to the invention may be prepared in various ways to suit specific applications.

The Example 2 studies demonstrate yet another way in which anaesthetic bone cement compositions according to the invention may be prepared to suit specific applications. Specifically, the type of bone cement employed can be used to control the characteristics of the release of anaesthetic. For example, the studies show that, when employing DePuy CMW3, crystalline lidocaine base, crystalline lidocaine HCl, and liquid prilocaine base, are eluted in greater quantities both initially and after 72 hours, than when employing Depuy CMW Endurance bone cement. Therefore, as discussed above with respect to the effect of particle size on anaesthetic release profiles, the use of a specific brand of bone cement in combination with a specific anaesthetic can provide an anaesthetic bone cement with a desired release profile. The profile can be optimized, for example, for fast or slow initial release, as required for a specific therapeutic application. Suitable combinations of bone cement and anaesthetic would be determined through routine experimentation using techniques described in the below examples. Although studies involving bone cement from other manufacturers are not described in Example 2, it is expected that other combinations of bone cement and forms of anaesthetic would similarly provide different release profiles suitable for a range of applications.

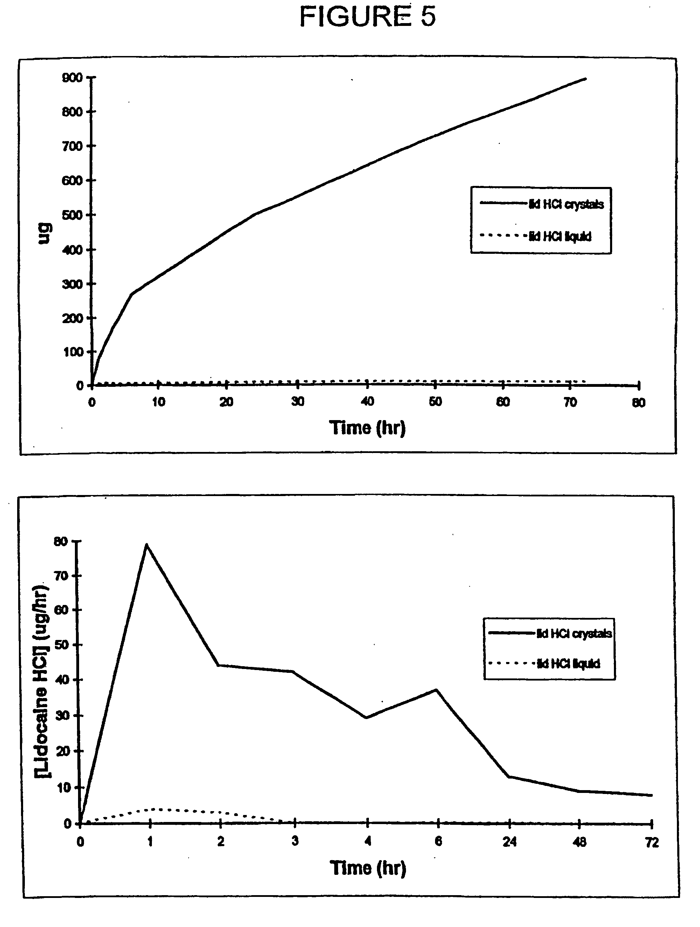

The studies of Example 2 further indicate that the choice of acid or base form of an anaesthetic can substantially affect how much of the anaesthetic is released from the bone cement. For example, when crystalline lidocaine is combined with DePuy CMW3 bone cement, there is substantially more total release of the anaesthetic after 72 hours if lidocaine HCl is used. Similarly, the physical form of anaesthetic is a variable that significantly affects the release of anaesthetic when combined with, for example, DePuy CMW3 bone cement. For example, as shown in Figure 5, there is substantial release of crystalline lidocaine HCl, and only a small release of liquid lidocaine HCl. It is apparent from these studies that the opposite applies for prilocaine base, wherein release of the liquid form was superior to that of the crystalline form. It is again evident that a desired release profile of an anaesthetic bone cement according to the invention can be designed by selecting, for example, a base or HCl, or a liquid or crystalline, form of an anaesthetic in combination with a suitable bone cement. The invention also contemplates the incorporation of a combination of anaesthetics with different properties into bone cement. Such a combination would comprise, for example, two or more different anaesthetics (e.g., lidocaine and prilocaine), or two or more different forms of an anaesthetic (e.g. HCl and base, or crystalline and liquid). Provision of such a combination of anaesthetics into bone cement would allow the release profile of anaesthetics, and hence profile of the analgesia produced thereby, to be further customized for particular therapeutic applications. All such combinations are considered to be within the scope of the present invention.

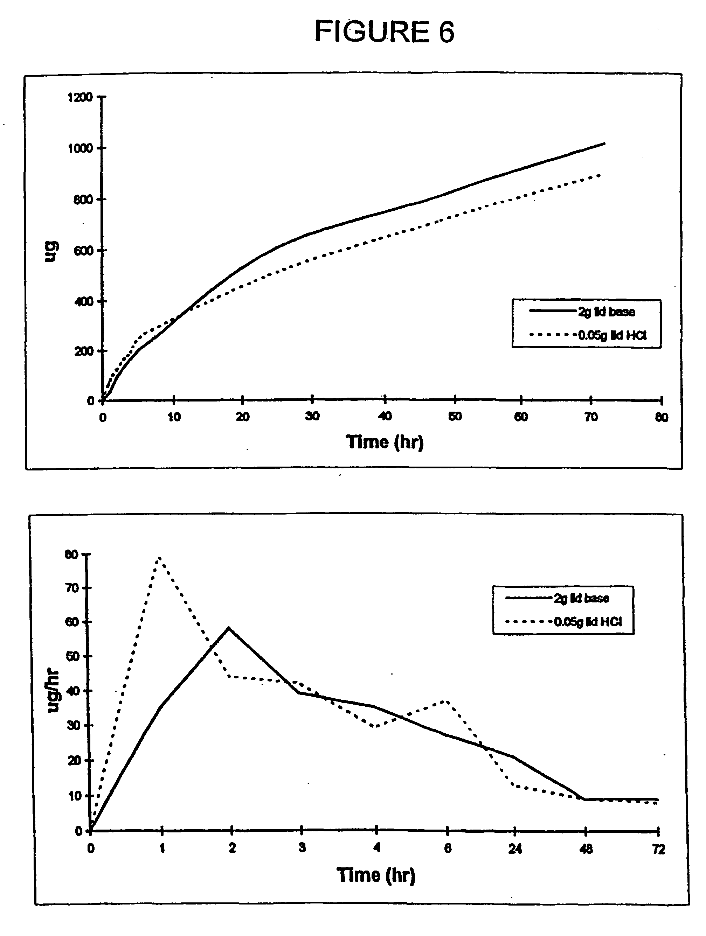

Thus, the studies of Example 2 clearly demonstrate that particular combinations of bone cement and anaesthetic may advantageously be chosen to achieve a desired result. The primary advantage of choosing a combination with superior release of anaesthetic is that less anaesthetic need be used. This is exemplified by the comparison below of 2.0 g lidocaine base with 0.05 g lidocaine HCl, from which it can be seen that 40 times less lidocaine HCl is required to achieve the same result as that achieved with lidocaine base (Figure 6). Indeed, as shown in Example 2, efficacious amounts of crystalline lidocaine HCl are released from bone cement when 0.005 g of lidocaine are combined with 57.9 g wet bone cement (i.e., 0.0086% by weight of bone cement). The invention thus encompasses the use of potent, i.e., highly releasable, forms of anaesthetics in combination with bone cement. Further, the invention contemplates the use of compounds for increasing the potency or releasability of anaesthetics from bone cement.

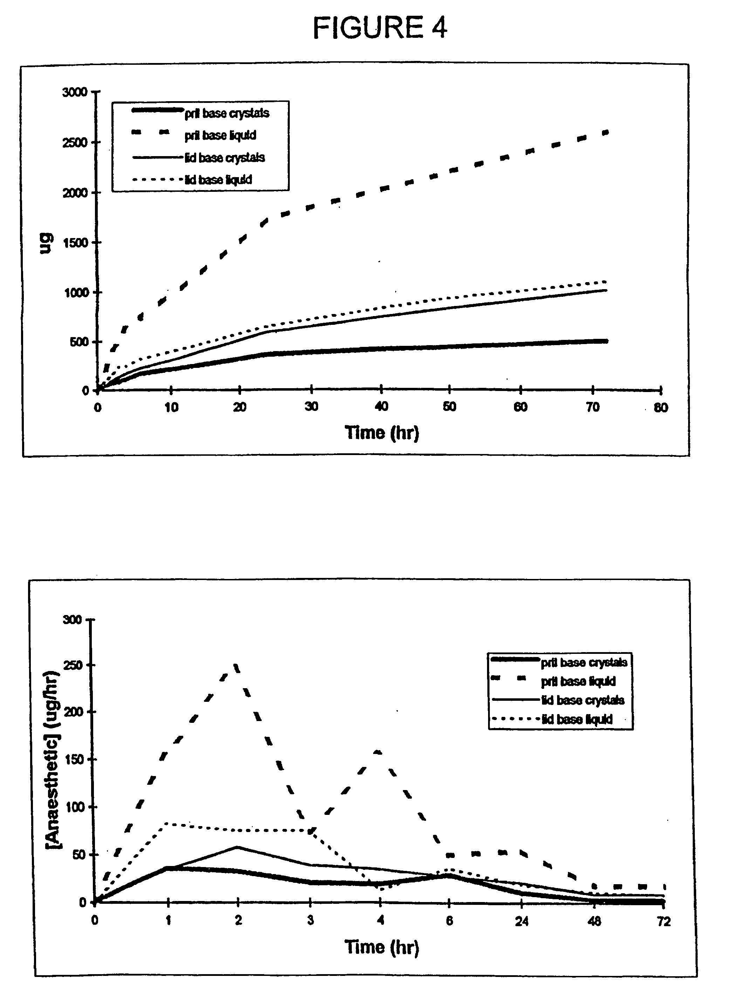

A further advantage of choosing a particular combination as taught by the invention is that, in combinations where release of anaesthetic is greater initially, the desired efficacy (i.e. pain reduction or elimination) is achieved within a shorter period following application of the bone cement and anaesthetic combination. For example, elution of liquid prilocaine base from CMW3 bone cement is initially greater than that of its crystalline counterpart as well as either form of lidocaine base (see Figure 4). On the other hand, where a slower initial release of anaesthetic is desired, use of crystalline prilocaine base, for example, in CMW3 bone cement would be suitable.

That the compositions, process, and use of the invention are efficacious in clinical applications can be seen from the results of a knee replacement surgery described in Example 3. From this example, it is apparent that lidocaine is eluted from Howmedica bone cement and within 6 hours of the application of the bone cement, sufficient lidocaine has eluted to provide an analgesic effect which persists for at least 24 hours and probably at least several days before the anaesthetic is metabolized in the body.

An additional study was undertaken to address the question of whether the incorporation of an anaesthetic into bone cement according to the invention produces any undesirable effects on the mechanical properties of bone cement (see Example 4). Lidocaine (Xylocaine, Astra Pharmaceuticals) was combined with DePuy CMW3 bone cement as described in Example 1, and mechanical properties including dough time, setting time, exotherm, compressive strength, flexural strength, flexural modulus, and impact strength were analyzed.

As can be seen from Table 1, the incorporation of lidocaine into bone cement had no negative effects on the mechanical properties of the bone cement. In fact, there was a small increase in impact strength and an increase in dough time and setting time, which increases may be considered advantageous under certain circumstances.

It will, of course, be appreciated that other proprietary bone cements can equally well be employed, such as, for example, Palacos®R which is distributed by Schering Plough in Europe and by Richards in North America. The invention also contemplates incorporation of local anaesthetics into proprietary bone wax compositions, such as Ethicon® Bone Wax, which is a sterile mixture of beeswax and isopropyl palmitate, a wax softening used to control bleeding from bone surfaces. The invention further contemplates incorporation of local anaesthetics into injectable bone substitutes, or bone paste, such as Norian Skeletal Repair System (Norican SRS™) developed by Norian Corporation of Cupertino, CA, which is a calcium phosphate based cement which, when injected, forms carbonated apatite.

### Example 1

### Method

Forty grams of bone cement powder from each of three manufacturers: Howmedica (Simplex), Zimmer (Osteobond) and DePuy (CMW3), were mixed with 0.5, 1.0 and 2.0 g of gas-sterilized crystalline lidocaine base (Xylocaine, Astra Pharmaceuticals). The polymerization initiated mixtures were formed into discs 50 mm x 1 mm and allowed to harden. The hardened discs were then placed in a stirred solution (100 ml) containing 0.2% saline at 37°C. 100 µl aliquots were taken at 1, 2, 3, 4, 6, 24, 48 and 72 hours and subjected to HPLC with electrochemical detection analysis to determine the lidocaine level in each sample.

### Results

Typical elution profiles are shown in Figures 1 (Howmedica), 2 (Zimmer) and 3 (DePuy). These profiles demonstrate that lidocaine elutes from the bone cement mixture in an amount proportional to the amount of lidocaine in the mixture. The rate of elution is at a maximum during the first 24 hours and then tapers off. The curves also indicate that there is a peak dose at about the 6 hour point. The peak dose then provides sustained release over a 72-hour test period. It also appears that elution occurs mainly from the surface of the disc and is related to the porosity and other surface properties of the disc.

### Example 2

### Method

Crystalline lidocaine base from three manufacturers (Sigma, Spectrum, and Wyckoff) was used in these studies. It was observed that the relative proportions of crystals of different size varied substantially among the three brands. Thus, an initial study compared the effect of crystal size on elution and release characteristics using Spectrum lidocaine base. Crystals were sorted using a series of molecular sieves into three groups (small, 75 to 150 um; medium, 150 to 250 um; and large, >250 um) and 2.0 g samples from each group were incorporated into bone cement and tested, as indicated below.

All studies adhered to the following procedure: Forty grams of bone cement (DePuy CMW3 unless otherwise specified) were mixed with 2.0, 0.5, 0.05, or 0.005 g of liquid or crystalline anaesthetic (lidocaine: Sigma, Spectrum, Wyckoff; prilocaine: Colour Your Enzyme, Queen's University, Kingston, Ontario, Canada). The polymerization initiated mixtures were formed into discs 50 mm x 1 mm and allowed to cure. The cured discs were then placed in a solution (100 ml) containing 0.2% saline, and kept at 37°C in a shaking water bath. 1 ml aliquots were taken at 0, 1, 2, 3, 4, 6, 24, 48 and 72 hours and to each was added 1 µg/ml bupivacaine as an internal standard. 50 µl aliquots were subjected to HPLC using a Beckman reverse phase column having a mobile phase of 40 mM ammonium phosphate:acetonitrile (60:40) and a flow rate of 1 ml/min, followed by electrochemical detection, to determine the amount of anaesthetic in each sample.

The percentage of anaesthetic released was calculated from the mass of the bone cement/anaesthetic mixture: anaesthetic (e.g., 0.05 g) + bone cement powder (40 g) + bone cement liquid (17.9 g) = 57.95 g. The mean mass of bone cement discs in an individual experiment was, for example, 3.75 g, which corresponds to 15.45 discs per mixture. For elution of, for example, 1000 ug after 72 hours, 1000 ug X 15.45 discs = 0.015 g anaesthetic released. Since 0.05 g anaesthetic was used, 30.9% was released.

Studies examined the effects of manufacturer of anaesthetic, type of anaesthetic (i.e., lidocaine vs. prilocaine), physical form (i.e., crystalline vs. liquid), particle size, type of bone cement, concentration of anaesthetic, and chemical form (i.e., base vs. HCl) on release profiles (i.e., the amount of anaesthetic released after a given period and the change in rate of elution of anaesthetic over time).

### Results

### Manufacturer of lidocaine base

Crystalline lidocaine base produced by the three manufacturers had similar elution profiles. In each case, about 1% of the 2.0 g of lidocaine was released over 72 hours.

### Prilocaine base vs. lidocaine base:

As shown in Figure 4, elution and release of crystalline forms of both anaesthetics were the same, with about 0.4 % and 1 % of the 2.0 g of each of prilocaine and lidocaine, respectively, being released. Initial release of anaesthetic was greatest for liquid prilocaine base. Further, about 2 % of liquid prilocaine base was released, while only 1 % of liquid lidocaine base was released.

### Crystalline vs. liquid anaesthetic:

*Lidocaine base:* Elution characteristics of crystalline and liquid lidocaine base were the same. In both cases, about 1% of the 2 g of lidocaine was released over 72 hours.

*Lidocaine HCl*: As shown in Figure 5, about 25 % of the 0.05 g of crystalline lidocaine HCl was released from CMW3 bone cement. In contrast, only 0.3 % of the 0.05 g sample of liquid lidocaine HCl was released.

*Prilocaine base*: There was a difference between crystalline and liquid prilocaine, wherein 0.4 % of the 2.0 g of crystals, and 2 % of the 2.0 g of liquid was released.

### Particle size

*Lidocaine base*: There was no effect of particle size on the release profile or overall release of lidocaine base. In all size groups (75 to 150 um, 150 to 250 um, and >250 um), about 1% of the 2.0 g of lidocaine used was released over the 72 hour period.

*Lidocaine HCl*: Smaller particles eluted faster, such that elution during the latter portion of the 72 hour period was predominantly from larger particles. Overall, 30 to 34 % of the lidocaine of the 0.05 g samples was released for all three ranges of particle size. Similar results were obtained with 0.005 g samples, with 17 to 26 % overall release.

### DePuy CMW3 bone cement vs. DePuy CMW Endurance bone cement:

*Lidocaine base:* Release of crystalline lidocaine base from CMW3 bone cement was about 1 % after 72 h in CMW3, and only about 0.3 % after 72 h in CMW Endurance.

*Lidocaine HCl*: A similar effect of type of bone cement was obtained when 0.5 g samples of crystalline lidocaine HCl were used, wherein about 33 % was released from CMW3, and 23 % from CMW Endurance.

*Prilocaine base*: Release of crystalline prilocaine base from both types of bone cement was similar, and about 0.4 % of the 2.0 g of prilocaine was released. However, substantially more liquid prilocaine base was released from CMW3 than from CMW Endurance (2 % vs about 0.3 %).

### Concentration of crystalline lidocaine HCl:

There was no apparent substantive difference in elution profile of three concentrations of lidocaine HCl (0.5, 0.05, and 0.005 g per 40 g bone cement). At 0.5 g, release was about 33 %; at 0.05 g, about 25 %; and at 0.005 g, 17 to 26 %.

### Lidocaine base vs. lidocaine HCl:

Comparison of 2.0 g lidocaine base with 0.05 g lidocaine HCl indicated that the two forms of lidocaine had similar release profiles (see Figure 6). However, because of the differences in release, approximately 40 times less anaesthetic is needed to achieve the same effect if lidocaine HCl is used, rather than lidocaine base.

### Example 3

A female patient, 68 years old, having a previous total knee replacement and a below-the-knee amputation, presented with latent infection in the knee. A revision to remove the knee prosthesis was performed and the cut ends of the bone were treated with an anti-bacterial bone cement to keep the bones spaced. Three weeks later, the bone cement was removed and tissue samples were taken for laboratory analysis for signs of infection. Bone cement was temporarily applied to keep the bones spaced and aligned, but this time the cement was Howmedica bone cement containing 2 g of lidocaine (Xylocaine) per 40 g package of cement. The lidocaine-containing bone cement was gas sterilized but not irradiated. After recovery from anaesthesia the patient reported severe pain in the knee for a period of approximately 6 hours and thereafter no pain at all. 24 hours post surgery, the patient was sleeping without the aid of pain killers and was also able to receive physiotherapy without feeling undue discomfort.

### Example 4

The effects of lidocaine (Xylocaine) on the mechanical properties of Depuy CMW3 bone cement were evaluated and are summarized in Table 1 below.

**TABLE 1**

| **Cement Property** | **CMW3** | **CMW3 + Lidocaine** |
|---|---|---|
| Dough time (min:sec) | 2:50 | 7:05 |
| Setting time (min:sec) | 10:06 | 14:20 |
| Exotherm(°C) | 70.8 | 69.1 |
| Compressive strength (Mpa) | 112.0 | 113.3 |
| Flexural strength (Mpa) | 65.0 | 66.3 |
| Flexural modulus (Mpa) | 2785 | 2753 |
| Impact strength (J/m) | 3.03 | 3.61 |

From Table 1 it can be seen that addition of lidocaine to CMW3 improves impact strength by about 10%, but has little effect on compressive strength, flexural strength or flexural modulus. It is particularly noted that lidocaine additions increase the cement setting time by about 40% and the "doughing time," i.e., the time needed to reach a working mix that can be readily handled, by a factor of 3.

## Claims

1. An anaesthetic bone cement composition comprising:
(a) an acrylic bone cement; and
(b) an amount up to about 5% by weight of a local anaesthetic agent;
wherein at least a portion of the anaesthetic agent is released from said bone cement composition when the bone cement composition is in a subject and the strength of the bone cement is substantially unchanged.

2. An anaesthetic bone cement composition according to Claim 1 wherein said bone cement comprises:
(a) a liquid monomeric (meth)acrylate composition; and
(b) a powder comprising at least one of a homopolymer and a copolymer of methyl methacrylate containing an effective amount of a polymerization initiator.

3. An anaesthetic bone cement composition according to Claim 1 or Claim 2 wherein said local anaesthetic is selected from lidocaine, bupivacaine,prilocaine and tetracaine.

4. An anaesthetic bone cement composition according to any one of Claims 1 to 3 additionally including a radiopacifier.

5. An anaesthetic bone cement composition according to any one of Claims 1 to 4 wherein said local anaesthetic is in a physical form selected from liquids and solids.

6. An anaesthetic bone cement composition according to any one of Claims 1 to 4 wherein said local anaesthetic is in a chemical form selected from acids and bases.

7. An anaesthetic bone cement composition according to any of the preceding Claims, wherein the local anaesthetic is released from the bone cement in a preselected characteristic release profile that is determined by at least one of chemical form and physical form of the anaesthetic.

8. An anaesthetic bone cement composition according to any of the preceding Claims wherein said anaesthetic is present in an amount between about 0.007% and about 5% by weight of said cement composition.

9. An anaesthetic bone cement composition according to any of the preceding Claims wherein the local anaesthetic is provided in at least two different forms, the forms being selected from acids, bases, solids and liquids.

10. An anaesthetic bone cement composition according to any of the preceding Claims wherein the local anaesthetic comprises a combination of at least two anaesthetics selected from lidocaine, bupivacaine, prilocaine and tetracaine.

11. An anaesthetic bone cement composition according to Claim 10 wherein the anaesthetics included in the combination of at least two local anaesthetics are provided in at least two different forms, the forms being selected from acids, bases, solids, and liquids.

12. An anaesthetic bone cement composition according to Claim 10 wherein the anaesthetics included in the combination of at least two local anaesthetics are provided in the same form, the form being selected from acids, bases, solids, and liquids.

13. An anaesthetic bone cement composition according to any one of Claims 2 to 12 wherein said polymerization initiator is an organic peroxy compound.

14. An anaesthetic bone cement composition according to Claim 13 wherein said peroxy compound is benzoyl peroxide

15. A process for producing an anaesthetic bone cement composition comprising combining a bone cement with an effective amount up to about 5% by weight of a local anaesthetic.

16. A process for producing an anaesthetic bone cement composition according to Claim 15 comprising combining: (a) a liquid monomeric (meth)acrylate; (b) a powdered component comprising at least one of a homopolymer and a copolymer of methyl methacrylate, an effective amount of a polymerization initiator and a radiopacifier; and (c) said local anaesthetic.

17. A process according to Claim 15, including the step of gas sterilizing said bone cement.

18. A process according to any one of Claims 15-17 wherein the local anaesthetic is selected from lidocaine, bupivacaine, prilocaine and tetracaine.

19. A process according to Claim 18 wherein the local anaesthetic is in a physical form selected from liquids and solids.

20. A process according to Claim 18 wherein the local anaesthetic is in a chemical form selected from acids and bases.

21. A process according to any one of Claims 15-20 wherein the local anaesthetic is released from the bone cement in a preselected characteristic release profile that is determined by at least one of chemical form and physical form of the anaesthetic.

22. A process according to any one of Claims 15-21 wherein the anaesthetic is present in an amount between 0.007% and 5% by weight of said cement composition.

23. A process according to Claim 21 wherein the local anaesthetic is provided in at least two different forms, the forms being selected from acids, bases, solids, and liquids.

24. A process according to Claims 15-17 wherein the local anaesthetic comprises a combination of at least two anaesthetics selected from lidocaine, bupivacaine, prilocaine, and tetracaine.

25. A process according to Claim 24 wherein the anaesthetics included in the combination of at least two local anaesthetics are provided in at least two different forms, the forms being selected from acids, bases, solids and liquids.

26. A process according to Claim 24 wherein the anaesthetics included in the combination of at least two local anaesthetics are provided in the same form, the form being selected from acids, bases, solids and liquids.

27. A process according to Claim 16 wherein the polymerisation initiator is an organic peroxy compound.

28. A process according to Claim 27 wherein the peroxy compound is benzoyl peroxide.

29. A composition comprising:
(a) an acrylic bone cement; and
(b) an amount up to about 5% by weight of a local anaesthetic agent; for use as an anaesthetic bone cement as claimed in any of Claims 1-14 for producing analgesia at an orthopaedic implant site in a patient.

30. Use of a composition comprising:
(a) an acrylic bone cement; and
(b) an amount up to about 5% by weight of a local anaesthetic agent;
for the manufacture of an anaesthetic bone cement as claimed in any of Claims 1-14 for producing analgesia at an orthopaedic implant site in a patient.

## Patentansprüche

1. Anästhetische Knochenzementzusammensetzung umfassend (a) einen Acrylknochenzement und (b) ein Lokalanästhetikum in einer Menge von bis zu etwa 5 Gew.-%, worin wenigstens ein Teil des Lokalanästhetikums aus der Knochenzementzusammensetzung freigesetzt wird, wenn sich die Knochenzementzusammensetzung in einem Patienten befindet und sich die Festigkeit des Knochenzements im Wesentlichen nicht verändert.

2. Anästhetische Knochenzementzusammensetzung nach Anspruch 1, worin der Knochenzement (a) eine flüssige monomere (Meth)acrylat-Zusammensetzung und (b) ein Pulver umfassend wenigstens ein Homopolymer und/oder ein Copolymer aus Methylmethacrylat, das eine wirksame Menge eines Polymerisationsstarters enthält, umfasst.

3. Anästhetische Knochenzementzusammensetzung nach Anspruch 1 oder 2, worin das Lokalanästhetikum aus Lidocain, Bupivacain, Prilocain und Tetracain ausgewählt ist.

4. Anästhetische Knochenzementzusammensetzung nach einem der Ansprüche 1 bis 3, die zusätzlich ein Kontrastmittel enthält.

5. Anästhetische Knochenzementzusammensetzung nach einem der Ansprüche 1 bis 4, worin das Lokalanästhetikum in einer aus Flüssigkeiten und Feststoffen ausgewählten physikalischen Form vorliegt.

6. Anästhetische Knochenzementzusammensetzung nach einem der Ansprüche 1 bis 4, worin das Lokalanästhetikum in einer aus Säuren und Basen ausgewählten chemischen Form vorliegt.

7. Anästhetische Knochenzementzusammensetzung nach einem der vorhergehenden Ansprüche, worin das Lokalanästhetikum mit einem vorgewählten charakteristischen Freisetzungsprofil, das durch die chemische Form und/oder die physikalische Form des Anästhetikums bestimmt ist, aus dem Knochenzement freigesetzt wird.

8. Anästhetische Knochenzementzusammensetzung nach einem der vorhergehenden Ansprüche, worin das Anästhetikum in einer Menge zwischen etwa 0,007 Gew.-% und etwa 5 Gew.-% der Zementzusammensetzung vorliegt.

9. Anästhetische Knochenzementzusammensetzung nach einem der vorhergehenden Ansprüche, worin das Lokalanästhetikum in wenigstens zwei verschiedenen Formen bereitgestellt ist, wobei die Formen aus Säuren, Basen, Feststoffen und Flüssigkeiten ausgewählt sind.

10. Anästhetische Knochenzementzusammensetzung nach einem der vorhergehenden Ansprüche, worin das Lokalanästhetikum eine Kombination von wenigstens zwei aus Lidocain, Bupivacain, Prilocain und Tetracain ausgewählten Anästhetika umfasst.

11. Anästhetische Knochenzementzusammensetzung nach Anspruch 10, worin die in der Kombination von wenigstens zwei Lokalanästhetika enthaltenen Anästhetika in wenigstens zwei verschiedenen Formen bereitgestellt sind, wobei die Formen aus Säuren, Basen, Feststoffen und Flüssigkeiten ausgewählt sind.

12. Anästhetische Knochenzementzusammensetzung nach Anspruch 10, worin die in der Kombination von wenigstens zwei Lokalanästhetika enthaltenen Anästhetika in derselben Form bereitgestellt sind, wobei die Form aus Säuren, Basen, Feststoffen und Flüssigkeiten ausgewählt ist.

13. Anästhetische Knochenzementzusammensetzung nach einem der Ansprüche 2 bis 12, worin der Polymerisationsstarter eine organische Peroxyverbindung ist.

14. Anästhetische Knochenzementzusammensetzung nach Anspruch 13, worin die Peroxyverbindung Benzoylperoxid ist.

15. Verfahren zur Herstellung einer anästhetischen Knochenzementzusammensetzung umfassend das Vereinigen eines Knochenzements mit einer wirksamen Menge von bis zu etwa 5 Gew.-% eines Lokalanästhetikums.

16. Verfahren zur Herstellung einer anästhetischen Knochenzementzusammensetzung nach Anspruch 15 ,umfassend das Kombinieren (a) eines flüssigen monomeren (Meth)acrylats, (b) einer pulverförmigen Komponente, die ein Homopolymer und/oder ein Copolymer von Methylmethacrylat, eine wirksame Menge eines Polymerisationsstarters und ein Kontrastmittel enthält, und (c) des Lokalanästhetikums.

17. Verfahren nach Anspruch 15, das den Schritt des Gassterilisierens des Knochenzements beinhaltet.

18. Verfahren nach einem der Ansprüche 15 bis 17, worin das Lokalanästhetikum aus Lidocain, Bupivacain, Prilocain und Tetracain ausgewählt ist.

19. Verfahren nach Anspruch 18, worin das Lokalanästhetikum in einer aus Flüssigkeiten und Feststoffen ausgewählten physikalischen Form vorliegt.

20. Verfahren nach Anspruch 18, worin das Lokalanästhetikum in einer aus Säuren und Basen ausgewählten chemischen Form vorliegt.

21. Verfahren nach einem der Ansprüche 15 bis 20, worin das Lokalanästhetikum mit einem vorgewählten charakteristischen Freisetzungsprofil, welches durch die chemische Form und/oder die physikalische Form des Anästhetikums bestimmt ist, aus dem Knochenzement freigesetzt wird.

22. Verfahren nach einem der Ansprüche 15 bis 21, worin das Anästhetikum in einer Menge zwischen 0,007 Gew.-% und 5 Gew.-% der Zementzusammensetzung vorliegt.

23. Verfahren nach Anspruch 21, worin das Lokalanästhetikum in wenigstens zwei verschiedenen Formen bereitgestellt wird, wobei die Formen aus Säuren, Basen, Feststoffen und Flüssigkeiten ausgewählt sind.

24. Verfahren nach einem der Ansprüche 15 bis 17, worin das Lokalanästhetikum eine Kombination von wenigstens zwei aus Lidocain, Bupivacain, Prilocain und Tetracain ausgewählten Anästhetika umfasst.

25. Verfahren nach Anspruch 24, worin die in der Kombination aus wenigstens zwei Lokalanästhetika enthaltenen Anästhetika in wenigstens zwei verschiedenen Formen bereitgestellt werden, wobei die Formen aus Säuren, Basen, Feststoffen und Flüssigkeiten ausgewählt sind.

26. Verfahren nach Anspruch 24, worin die in der Kombination von wenigstens zwei Lokalanästhetika enthaltenen Anästhetika in derselben Form bereitgestellt werden, wobei die Form aus Säuren, Basen, Feststoffen und Flüssigkeiten ausgewählt ist:

27. Verfahren nach Anspruch 16, worin der Polymerisationsstarter eine organische Peroxyverbindung ist.

28. Verfahren nach Anspruch 27, worin die Peroxyverbindung Benzoylperoxid ist.

29. Zusammensetzung umfassend (a) einen Acrylknochenzement und (b) eine Menge von bis zu etwa 5 Gew.-% eines Lokalanästhetikums, zur Verwendung als anästhetischer Knochenzement gemäß einem der Ansprüche 1 bis 14, um eine Schmerzunempfindlichkeit an einer orthopädischen Implantationsstelle in einem Patienten zu erzeugen.

30. Verwendung einer Zusammensetzung umfassend (a) einen Acrylknochenzement, und (b) eine Menge von bis etwa 5 Gew.-% eines Lokalanästhetikums, um einen anästhetischen Knochenzement gemäß einem der Ansprüche 1 bis 14 zur Erzeugung von Schmerzunempfindlichkeit an einer orthopädischen Implantationsstelle in einem Patienten zu erzeugen.

## Revendications

1. Composition de ciment osseux anesthésique, comprenant :
(a) un ciment osseux acrylique ; et
(b) une quantité allant jusqu'à 5% environ en poids d'un agent anesthésique local ;
dans laquelle au moins une partie de l'agent anesthésique est libérée de ladite composition de ciment osseux lorsque la composition de ciment osseux est placée dans un sujet et la résistance du ciment osseux reste sensiblement inchangée.

2. Composition de ciment osseux anesthésique selon la revendication 1, dans laquelle ledit ciment osseux comprend :
(a) une composition de (méth)acrylate monomère liquide ; et
(b) une poudre contenant au moins l'un parmi un homopolymère et un copolymère de méthacrylate de méthyle contenant une quantité efficace d'un initiateur de polymérisation.

3. Composition de ciment osseux anesthésique selon la revendication 1 ou la revendication 2, dans laquelle ledit agent anesthésique local est choisi parmi la lidocaïne, la bupivacaine, la prilocaïne et la tétracaïne.

4. Composition de ciment osseux anesthésique selon l'une quelconque des revendications 1 à 3, contenant en outre un radio-opacifiant.

5. Composition de ciment osseux anesthésique selon l'une quelconque des revendications 1 à 4, dans laquelle ledit agent anesthésique local se présente sous une forme physique choisie parmi les liquides et les solides.

6. Composition de ciment osseux anesthésique selon l'une quelconque des revendications 1 à 4, dans laquelle ledit agent anesthésique local se présente sous une forme chimique choisie parmi les acides et les bases.

7. Composition de ciment osseux anesthésique selon l'une quelconque des revendications précédentes, dans laquelle l'agent anesthésique local est libéré du ciment osseux selon un profil de libération caractéristique présélectionné qui est déterminé par au moins l'une de la forme chimique et de la forme physique de l'agent anesthésique.

8. Composition de ciment osseux anesthésique selon l'une quelconque des revendications précédentes, dans laquelle ledit agent anesthésique est présent en une quantité comprise entre 0,007% environ et 5% environ en poids de ladite composition de ciment.

9. Composition de ciment osseux anesthésique selon l'une quelconque des revendications précédentes, dans laquelle l'agent anesthésique local est fourni sous au moins deux formes différentes, les formes étant choisies parmi les acides, les bases, les solides et les liquides.

10. Composition de ciment osseux anesthésique selon l'une quelconque des revendications précédentes, dans laquelle l'agent anesthésique local contient une combinaison d'au moins deux agents anesthésiques choisis parmi la lidocaïne, la bupivacaïne, la prilocaïne et la tétracaïne.

11. Composition de ciment osseux anesthésique selon la revendication 10, dans laquelle les agents anesthésiques contenus dans la combinaison d'au moins deux agents anesthésiques locaux sont fournis sous au moins deux formes différentes, les formes étant choisies parmi les acides, les bases, les solides et les liquides.

12. Composition de ciment osseux anesthésique selon la revendication 10, dans laquelle les agents anesthésiques contenus dans la combinaison d'au moins deux agents anesthésiques locaux sont fournis sous la même forme, la forme étant choisie parmi les acides, les bases, les solides et les liquides.

13. Composition de ciment osseux anesthésique selon l'une quelconque des revendications 2 à 12, dans laquelle ledit initiateur de polymérisation est un composé peroxyde organique.

14. Composition de ciment osseux anesthésique selon la revendication 13, dans laquelle ledit composé peroxyde est le peroxyde de benzoyle.

15. Procédé de fabrication d'une composition de ciment osseux anesthésique, comprenant un ciment osseux avec une quantité efficace allant jusqu'à 5% environ en poids d'un agent anesthésique local.

16. Procédé de fabrication d'une composition de ciment osseux anesthésique selon la revendication 15, comprenant la combinaison de : (a) un (méth)acrylate monomère liquide ; (b) un composant en poudre contenant au moins l'un parmi un homopolymère et un copolymère de méthacrylate de méthyle, une quantité efficace d'un initiateur de polymérisation et un radio-opacifiant ; et (c) ledit agent anesthésique local.

17. Procédé selon la revendication 15, comprenant l'étape consistant à stériliser par un gaz ledit ciment osseux.

18. Procédé selon l'une quelconque des revendications 15 à 17, dans lequel l'agent anesthésique local est choisi parmi la lidocaïne, la bupivacaïne, la prilocaïne et la tétracaïne.

19. Procédé selon la revendication 18, dans lequel l'agent anesthésique local se présente sous une forme physique choisie parmi les liquides et les solides.

20. Procédé selon la revendication 18, dans lequel l'agent anesthésique local se présente sous une forme chimique choisie parmi les acides et les bases.

21. Procédé selon l'une quelconque des revendications 15 à 20, dans lequel l'agent anesthésique local est libéré du ciment osseux selon un profil de libération caractéristique présélectionné qui est déterminé par au moins l'une de la forme chimique et de la forme physique de l'agent anesthésique.

22. Procédé selon l'une quelconque des revendications 15 à 21, dans lequel l'agent anesthésique est présent en une quantité comprise entre 0,007% et 5% en poids de ladite composition de ciment.

23. Procédé selon la revendication 21, dans lequel l'agent anesthésique local est fourni sous au moins deux formes différentes, les formes étant choisies parmi les acides, les bases, les solides et les liquides.

24. Procédé selon l'une quelconque des revendications 15 à 17, dans lequel l'agent anesthésique local contient une combinaison d'au moins deux agents anesthésiques choisis parmi la lidocaïne, la bupivacaïne, la prilocaïne et la tétracaïne.

25. Procédé selon la revendication 24, dans lequel les agents anesthésiques contenus dans la combinaison d'au moins deux agents anesthésiques locaux sont fournis sous au moins deux formes différentes, les formes étant choisies parmi les acides, les bases, les solides et les liquides.

26. Procédé selon la revendication 24, dans lequel les agents anesthésiques contenus dans la combinaison d'au moins deux agents anesthésiques locaux sont fournis sous la même forme, la forme étant choisie parmi les acides, les bases, les solides et les liquides.

27. Procédé selon la revendication 16, dans lequel l'initiateur de polymérisation est un composé peroxyde organique.

28. Procédé selon la revendication 27, dans lequel le composé peroxyde est le peroxyde de benzoyle.

29. Composition comprenant :
(a) un ciment osseux acrylique ; et
(b) une quantité allant jusqu'à 5% environ en poids d'un agent anesthésique local ; pour une utilisation en tant que ciment osseux anesthésique selon l'une quelconque des revendications 1 à 14, destiné à produire une analgésie au niveau d'un site d'implantation orthopédique chez un patient.

30. Utilisation d'une composition comprenant :
(a) un ciment osseux acrylique ; et
(b) une quantité allant jusqu'à 5% environ en poids d'un agent anesthésique local ;
pour la fabrication d'un ciment osseux anesthésique selon l'une quelconque des revendications 1 à 14, destiné à produire une analgésie au niveau d'un site d'implantation orthopédique chez un patient.
